# EUROPÄISCHE PATENTSCHRIFT

(11) **EP 3 287 157 B1**
(45) Veröffentlichungstag und Bekanntmachung des Hinweises auf die Patenterteilung: **14.08.2024**
(21) Anmeldenummer: 17187119.7
(22) Anmeldetag: 21.08.2017
(51) Int. Cl.: A61M 1/16

(54) **VERFAHREN UND VORRICHTUNG ZUR STERILISATION EINES DIALYSATORS**
METHOD AND DEVICE FOR STERILIZING A DIALYSER
PROCÉDÉ ET DISPOSITIF DE STÉRILISATION D'UN HÉMODIALYSEUR

(30) Priorität: 22.08.2016 DE 102016115498
(43) Veröffentlichungstag der Anmeldung: 28.02.2018
(73) Patentinhaber: B. Braun Avitum AG, 34212 Melsungen (DE)
(72) Erfinder: STARKE, Christian, 01454 Radeberg (DE); BURKERT, Sina, 01157 Dresden (DE)
(74) Vertreter: Winter, Brandl - Partnerschaft mbB

(56) Entgegenhaltungen:
- DE-U1- 202011 105 738
- US-A- 5 462 644
- US-A1- 2002 168 456
- US-A1- 2004 022 669
- US-A1- 2010 112 151

## Beschreibung

Die Erfindung betrifft ein Verfahren und eine Vorrichtung zur Sterilisation eines Dialysators für eine extrakorporale Blutbehandlung, und bezieht sich insbesondere auf ein Verfahren und eine Vorrichtung zur Sterilisation eines solchen Dialysators mittels eines gepulsten elektrischen Felds.

Bei der Herstellung von Dialysatoren ist deren korrekte und ausreichende Sterilisation wichtig, um Mikroorganismen mit schädlichen Wirkungen für einen Patienten, wie etwa Bakterien, Viren, Pilze und dergleichen, sicher abzutöten.

Gemäß der Europäischen Norm (EN) 556 kann ein Gegenstand dann als steril betrachtet werden, wenn der theoretische Wert von nicht mehr als einem lebenden Mikroorganismus in 1 × 10⁶ sterilisierten Einheiten des Endproduktes vorhanden ist. Für ausreichende Sterilität von Dialysatoren für extrakorporale Blutbehandlungen ist daher beispielsweise das Vorhandensein lebensfähiger Organismen um 6 Stufen auf ein Sterilisationsziel der Sterilisationssicherheitsebene (SAL; Sterility Assurance Level) 10⁻⁶ bzw. SAL10-6 zu reduzieren.

Gängige Verfahren zur Sterilisation von Dialysatoren beruhen beispielsweise auf der Nutzung oder Verwendung von Gammastrahlung, Elektronenstrahlung, Ethylenoxid (EtO/EO) und Heißdampf. In diesen Verfahren sind Strahlung, Hitze und Toxizität die auf Mikroorganismen abtötend wirkenden Effekte.

Alle der wie vorstehend bekannten Sterilisationsverfahren für Dialysatoren weisen allerdings spezifische Nachteile auf.

Die Gammasterilisation beispielsweise ist auf ein radioaktives Isotop (⁶⁰Co oder ¹³⁷Cs) angewiesen. Die Beschaffung desselben ist schwierig, der Transport ist aufwendig und es wird eine geeignete Endlagerstätte benötigt. Gammabestrahlungsanlagen erfordern zudem sehr aufwendige Maßnahmen zur Abschirmung der radioaktiven Strahlung.

Außerdem dauert der Bestrahlungsprozess aufgrund der prinzipbedingt geringen Dosisleistung mehrere Stunden.

Die Sterilisation mit Elektronenstrahlung benötigt zwar kein radioaktives Isotop, aber aufgrund beim Prozess entstehender Röntgenbremsstrahlung analog zur Gammasterilisation aufwendige Abschirmungsmaßnahmen. Des Weiteren sind zur Erzeugung von Elektronenstrahlen aufwendige und dementsprechend teure Beschleuniger notwendig, um die benötigte Eindringtiefe zu erreichen, die bei Elektronenstrahlen wesentlich geringer ist als bei Gammastrahlen.

Das für die EtO-Sterilisation verwendete Ethylenoxid wiederum ist ein starkes Protoplasmagift, kanzerogen, mutagen, allergisierend und chemisch-irritativ und daher hochgiftig, krebserregend und in einem Gasgemisch mit Luft mit einem Zündpunkt bei nur 40° außerdem hochexplosiv. Es stellt damit ein erhebliches Risiko dar. Das Entfernen des Ethylenoxids aus dem Dialysator nach erfolgter Sterilisation nimmt erhebliche Zeit in Anspruch.

Der zur Heißdampfsterilisation benötigte heiße Dampf schließlich muss energieaufwendig erzeugt werden und stellt aufgrund der hohen Temperaturen eine erhebliche Belastung für die Komponenten des Dialysators dar. Um die Heißdampfsterilisation anwenden zu können, muss der Dialysator außerdem speziellen Designanforderungen genügen. Das Dokument DE202011105738U offenbart den nächstliegenden Stand der Technik.

Vor diesem Hintergrund besteht Bedarf an alternativen Möglichkeiten zur Sterilisation speziell von Dialysatoren für extrakorporale Blutbehandlungen.

Der Erfindung liegt daher als eine Aufgabe zugrunde, ein alternatives und innovatives Sterilisationsverfahren zu schaffen, das die vorstehend genannten Nachteile der bekannten Sterilisationsverfahren überwindet und die Nutzung von gepulsten elektrischen Feldern (PEF) zur Sterilisation von Dialysatoren für die extrakorporale Blutbehandlung ermöglicht.

Erfindungsgemäß wird diese Aufgabe durch eine Vorrichtung mit den Merkmalen des Anspruchs 1 und durch ein Verfahren mit den Merkmalen des Anspruchs 12 gelöst. Ausführungsbeispiele, bei denen es einen physischen Kontakt der Einrichtung zur Erzeugung eines gepulsten elektrischen Felds mit einer in den Dialysator eingebrachten Flüssigkeit gibt, sind nicht Teil der Erfindung.

Vorteilhafte Weiterbildungen der Erfindung sind Gegenstand der beigefügten Unteransprüche.

In Übereinstimmung mit einer allgemeinen erfinderischen Idee zielt die Erfindung darauf ab, im Dialysator bereits vorhandene Keime sicher abzutöten. In anderen Worten soll der Kern der Erfindung nicht in einer Sterilisation eines Medizinprodukts oder in einem Verhindern eines Eindringens von Keimen in einen Dialysator bestehen, sondern in einer hinreichenden Abtötung von bereits in einem Dialysator vorhandener Verkeimung unter Erreichung jeweils erforderlicher SAL-Werte.

Dazu wird mittels einer Elektroporation durch ein elektrisches Feld, das beispielsweise als kurzer Puls durch den Entladungsstrom eines Kondensators erzeugt werden kann, die Zellmembran von Zellen aufgrund verschiedener Effekte permeabilisiert. Durch die temporäre Permeabilisierung kommt es zur Freisetzung von intrazellulären Bestandteilen, induziert durch hydrostatische Druckunterschiede und osmotische Effekte. Außerdem können Substanzen aus dem Außenmedium in das Zellinnere aufgenommen werden.

Diese Elektroporation kann zur Inaktivierung oder Abtötung von Mikroorganismen verwendet werden. Für ein zu behandelndes Objekt (Dialysator) wird dazu an diesem oder in der Umgebung desselben ein Reaktionsraum aufgebaut, in dem anhand eines oder mehrerer Elektrodenpaare ein gepulstes elektrisches Feld erzeugt wird. Die Wiederholungsrate der Pulse wird an einen vorliegenden Produktstrom angepasst. Für Mikroorganismen liegt eine wirksame elektrische Feldstärke in einem Bereich von 10 bis 40 kV/cm.

Durch gepulste elektrische Felder (Pulsed Electric Field, PEF) wie vorstehend beschrieben werden insbesondere die Zellmembranen von Bakterien und die DNS/RNS von Viren zerstört und diese somit abgetötet.

Gemäß der allgemeinen erfinderischen Idee werden somit ein Verfahren und eine Vorrichtung zur technischen Umsetzung des Verfahrens geschaffen, bei dem/der ein Dialysator mit gepulsten elektrischen Feldern (Pulsed Electric Field, PEF) sterilisiert wird, indem der Dialysator zunächst mit einer Flüssigkeit gefüllt wird und sodann über Elektroden Pulse elektrischen Stroms eingebracht werden, die eine Sterilisation der in den Dialysator eingebrachten Flüssigkeit und damit des Dialysators bewirken.

Die PEF-Sterilisation von Dialysatoren beruht dabei auf den beiden grundsätzlichen Annahmen oder Voraussetzungen, dass zum einen der Blutraum des Dialysators, d. h. alle Teile, die während einer Dialysebehandlung mit Blut in Berührung kommen, steril sein müssen, und zum anderen die Sterilisation einer in den Dialysator eingebrachten Flüssigkeit die Sterilisation des Blutraums des Dialysators bewirkt.

Dazu werden Elektroden auf jeweils zwei Seiten eines Dialysators derart angeordnet, dass der Innenraum des Dialysators (Schnittfläche, Faserinnenraum der Hohlfasern/Fasern) eine Sterilisationskammer bildet und auf diese Weise direkt die im Dialysator befindliche Flüssigkeit und damit auch direkt der Blutraum des Dialysators selbst sterilisiert wird.

Erfindungsgemäß ergeben sich durch die PEF-Sterilisation von Dialysatoren Vorteile dahingehend, dass nur ein geringer Energiebedarf (sehr hohe Spannungen, aber geringe Stromstärken und extrem kurze Zeitdauern (Pulse)) erforderlich ist, eine Sterilisation in sehr kurzer Zeit möglich ist, ein inline-Prozess, z. B. integrierbar in einen Faserintegritätstest, möglich ist, vorteilhaft insbesondere gegenüber radioaktiven Strahlungsquellen Abschaltbarkeit gegeben ist, und/oder eine Verwendung gefährlicher Stoffe wie etwa radioaktiver Isotope oder Ethylenoxid entfallen kann.

Im Einzelnen wird die Aufgabe gelöst durch eine Vorrichtung zur Sterilisation eines Dialysators für eine extrakorporale Blutbehandlung, beinhaltend eine Einrichtung zur Erzeugung eines gepulsten elektrischen Felds, die dazu angeordnet ist, bei Anlegen einer gepulsten elektrischen Spannung zwischen einer ersten Elektrode und einer zweiten Elektrode der Einrichtung ein den zwischen der ersten Elektrode und der zweiten Elektrode aufgenommenen Dialysator durchdringendes gepulstes elektrisches Feld zu erzeugen. Die Vorrichtung kann dazu vorteilhaft mehrteilig ausgebildet und für den Sterilisationsvorgang als Teil eines Vorbereitungs- oder Herstellungsprozesses des Dialysators vorübergehend mit dem Dialysator koppelbar sein, oder nach Art einer Festinstallation als definierter Raum entlang einer Prozesskette angeordnet sein, durch welchen der Dialysator während des Vorbereitungs- oder Herstellungsprozesses hindurchführbar ist oder in welchen der Dialysator vorübergehend einbringbar ist.

Bevorzugt weist die Einrichtung zur Erzeugung eines gepulsten elektrischen Felds einen ersten Abschnitt, der an einer ersten Stirnseite des Dialysators angeordnet ist bzw. daran angeordnet werden kann, und einen zweiten Abschnitt, der dem ersten Abschnitt gegenüberliegend an einer zweiten Stirnseite des Dialysators angeordnet ist bzw. daran angeordnet werden kann, auf, und sind die erste Elektrode in dem ersten Abschnitt und die zweite Elektrode in dem zweiten Abschnitt derart angeordnet bzw. können diese derart angeordnet werden, dass das gepulste elektrische Feld zumindest ein in dem Dialysator aufgenommenes Hohlfaserbündel in dessen Längsrichtung durchdringt. In dieser vorteilhaften Ausführungsform sind Flüssigkeitszulauf und Flüssigkeitsauslauf sowie Elektrode, ggf. umgeben von oder vergossen mit elektrischer Isolation, in die erforderlichen Anschlüsse bereitstellenden, kappenartigen Komponenten aufgenommen, die für zumindest die Dauer des Sterilisationsvorgangs an dem Dialysator festlegbar sind.

Bevorzugt sind die erste Elektrode und die zweite Elektrode hohlzylinderförmig erzeugt, weist der erste Abschnitt der Einrichtung zur Erzeugung eines gepulsten elektrischen Felds einen elektrischen Isolator auf, in dem die erste Elektrode aufgenommen ist, weist der zweite Abschnitt der Einrichtung zur Erzeugung eines gepulsten elektrischen Felds einen elektrischen Isolator auf, in dem die zweite Elektrode aufgenommen ist, und verläuft durch den ersten Abschnitt und durch den zweiten Abschnitt der Einrichtung zur Erzeugung eines gepulsten elektrischen Felds hindurch jeweils ein in den Dialysator und zur Außenseite der Einrichtung zur Erzeugung eines gepulsten elektrischen Felds hin öffnender Flüssigkeitskanal. Vorteilhaft kann hierdurch die benötigte Flüssigkeit stirnseitig des Dialysators zu- und abgeführt werden.

Bevorzugt verläuft der in den Dialysator öffnende Flüssigkeitskanal in Richtung der Längsachse des Dialysators durch den Isolator und durch den Hohlzylinder der Elektrode hindurch, wobei der Flüssigkeitskanal durch den Isolator gegenüber der Elektrode isoliert ist.

Alternativ bevorzugt sind die erste Elektrode und die zweite Elektrode scheibenförmig erzeugt, weist der erste Abschnitt der Einrichtung zur Erzeugung eines gepulsten elektrischen Felds einen elektrischen Isolator auf, in dem die erste Elektrode aufgenommen ist, weist der zweite Abschnitt der Einrichtung zur Erzeugung eines gepulsten elektrischen Felds einen elektrischen Isolator auf, in dem die zweite Elektrode aufgenommen ist, und verläuft durch den ersten Abschnitt und durch den zweiten Abschnitt der Einrichtung zur Erzeugung eines gepulsten elektrischen Felds hindurch jeweils ein in den Dialysator und zur Außenseite der Einrichtung zur Erzeugung eines gepulsten elektrischen Felds hin öffnender Flüssigkeitskanal.

Bevorzugt verläuft in der vorstehenden Alternative der in den Dialysator öffnende Flüssigkeitskanal auf einer ersten Länge in Richtung der Längsachse des Dialysators und auf einer zweiten Länge in Richtung der Querachse des Dialysators zwischen dem Dialysator und der Elektrode durch den Isolator hindurch. Vorteilhaft kann hierdurch die benötigte Flüssigkeit seitlich des Dialysators zu- und abgeführt werden.

Es versteht sich, dass die vorgenannten Alternativen kombinierbar sind, d.h. jeweils ein Flüssigkeitskanal auf einer Seite des Dialysators stirnseitig und auf einer anderen Seite des Dialysators an der Seite desselben vorgesehen sein kann. Hierdurch ergeben sich vorteilhaft zusätzliche Freiheitsgrade für beispielsweise unterschiedliche Dialysatoren und/oder Prozessumgebungen.

Bevorzugt ist die Einrichtung zur Erzeugung eines gepulsten elektrischen Felds mittels einer verschraubbaren Gewindeanordnung an dem Dialysator fluiddicht festlegbar.

Alternativ bevorzugt sind die Einrichtung zur Erzeugung eines gepulsten elektrischen Felds und der Dialysator über eine Dichtfläche, die im Bereich einer Schnittfläche ses Hohlfaserbündels das Hohlfaserbündel innenseitig der Wandung des Dialysators umläuft, und eine gegen die Dichtfläche anpressbare Erhebung fluiddicht verbindbar. Eine druckbasierte Kopplung einer Pulsfelderzeugungseinrichtung und des Dialysators erleichtert vorteilhaft die Automatisierung des Sterilisationsprozesses, da die Drehbewegung eines Verschraubungsvorgangs entfallen und die Kopplung in nur einer linearen Heranführ- und Wegführ-Bewegung erfolgen kann.

Alternativ bevorzugt weist die Einrichtung zur Erzeugung eines gepulsten elektrischen Felds einen ersten Abschnitt und einen zweiten Abschnitt, die einander gegenüberliegend entlang der Längsseite des Dialysators angeordnet sind, auf, und sind die erste Elektrode in dem ersten Abschnitt und die zweite Elektrode in dem zweiten Abschnitt derart angeordnet, dass das gepulste elektrische Feld zumindest das in dem Dialysator aufgenommene Hohlfaserbündel in dessen Querrichtung durchdringt. Die Anordnung der Elektroden entlang der Längsseite des Dialysators ermöglicht vorteilhaft eine Festinstallation eines Sterilisationsraums in der Prozesskette und die Vermeidung unnötiger Manipulation des Dialysators. Des Weiteren führt der im Vergleich zu den vorstehend erläuterten Ausführungsvarianten geringere Abstand der Elektroden zu einer Verringerung der zur Erreichung des Sterilisationsniveaus erforderlichen elektrischen Feldstärke und vereinfacht damit die Erzeugung derselben.

Bevorzugt sind in der vorgenannten Alternative die erste Elektrode und die zweite Elektrode plattenförmig eben ausgebildet und parallel zueinander angeordnet. Eine plattenförmig ebene Elektrodenform kann vorteilhaft und unabhängig von der Form des Dialysators eine präzise Vorbestimmung des zu erzeugenden elektrischen Felds erlauben.

Alternativ bevorzugt sind in der vorgenannten Alternative die erste Elektrode und die zweite Elektrode dem Umfang des Dialysators folgend gekrümmt ausgebildet. Eine gebogene Elektrodenform kann vorteilhaft eine präzise Abstimmung des zu erzeugenden elektrischen Felds auf die tatsächliche Dialysatorform erlauben.

Die Aufgabe wird auch gelöst durch ein Verfahren zur Sterilisation eines Dialysators für eine extrakorporale Blutbehandlung, mit den folgenden Schritten in einem Prozess zur Vorbereitung oder Herstellung des Dialysators: Erzeugen eines gepulsten elektrischen Felds mittels einer vorbestimmten Anzahl elektrischer Pulse einer definierten elektrischen Spannung, einer definierten Pulsdauer und einer definierten Pausenzeit zwischen den Pulsen, und Beaufschlagen des Dialysators mit dem gepulsten elektrischen Feld.

Bevorzugt beinhaltet das Verfahren ferner die Schritte: vor dem Erzeugen des gepulsten elektrischen Felds, Anordnen einer Einrichtung zur Erzeugung eines gepulsten elektrischen Felds auf jeweils beiden Seiten des Dialysators; und Füllen des Dialysators mit einer vorbestimmten Flüssigkeit vor dem Erzeugen des gepulsten elektrischen Felds; und nach Wegnahme des gepulsten elektrischen Felds, Verstreichenlassen einer vorbestimmten Einwirkungsdauer für durch die elektrischen Pulse erzeugte reaktive Substanzen; und Spülen des Dialysators zur Entfernung von Rückständen des Sterilisationsprozesses.

Bevorzugt wird das vorstehende Verfahren mit einer Vorrichtung einer der vorgenannten Ausführungsformen durchgeführt.

Die Erfindung wird nachstehend anhand bevorzugter Ausführungsbeispiele unter Bezugnahme auf die Zeichnung näher beschrieben. Es zeigen:
Fig. 1 schematisch eine Schnittansicht eines Dialysators mit einer PEF-Einrichtung, oberseitigem Flüssigkeitszulauf bzw. Flüssigkeitsablauf und Elektroden an einer Stirnseite des Dialysators gemäß einem ersten Ausführungsbeispiel der Erfindung;
Fig. 2 schematisch eine Schnittansicht eines Dialysators mit einer PEF-Einrichtung und seitlichem Flüssigkeitszulauf bzw. Flüssigkeitsablauf gemäß einem zweiten Ausführungsbeispiel der Erfindung;
Fig. 3 schematisch eine Modifikation des ersten und des zweiten Ausführungsbeispiels mit alternativer Abdichtung eines flüssigkeitsgefüllten Raums des Dialysators gemäß einem dritten Ausführungsbeispiel;
Fig. 4 schematisch eine Schnittansicht eines Dialysators mit einer PEF-Einrichtung, oberseitigem Flüssigkeitszulauf bzw. Flüssigkeitsablauf und Elektroden entlang der Längsseiten des Dialysators gemäß einem vierten Ausführungsbeispiel der Erfindung; und
Fig. 5 schematisch eine Aufsicht auf einen Dialysator mit einer PEF-Einrichtung und gekrümmtem Elektrodenverlauf gemäß einem fünften Ausführungsbeispiel.

Es wird angemerkt, dass in den Zeichnungen gleiche oder gleichwirkende Elemente und Komponenten mit denselben Bezugszeichen bezeichnet sind und nicht redundant beschrieben werden.

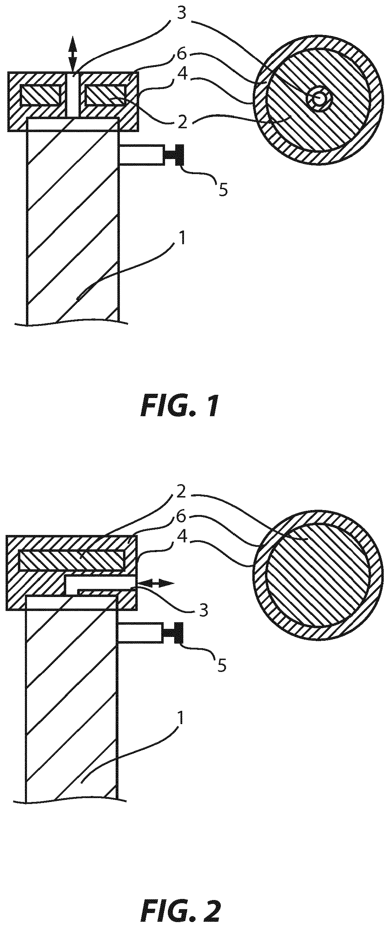

Fig. 1 zeigt schematisch eine Schnittansicht eines Dialysators 1 mit einer PEF-Einrichtung 4, einem oberseitigen Flüssigkeitszulauf bzw. Flüssigkeitsablauf 3 und zumindest zwei Elektroden 2 an einer Stirnseite des Dialysators gemäß einem ersten Ausführungsbeispiel. Ein seitlich angeordneter, sich im Dialysierflüssigkeitsstutzen befindlicher Stopfen ist mit dem Bezugszeichen 5 bezeichnet.

Die PEF-Einrichtung 4 ist eine Einrichtung zur Erzeugung eines gepulsten elektrischen Felds, die dazu angeordnet ist, durch Anlegen von elektrischen Pulsen vorbestimmter Form, Dauer und/oder Signalstärke (Spannung) mittels der Elektroden 2 ein den (flüssigkeitsgefüllten) Dialysator 1 in vorbestimmter Weise durchdringendes gepulstes elektrisches Feld zu erzeugen.

Der Dialysator 1 wird mit einer Flüssigkeit, vorzugsweise gereinigtes bzw. destilliertes Wasser vorbestimmter Reinheit, gefüllt. Die vorbestimmte Reinheit ist zweckmäßig, da es bei zu unreiner Lösung oder Flüssigkeit bei Anlegen des gepulsten elektrischen Felds zu einem unerwünschten Spannungsdurchschlag kommen kann. Der Flüssigkeit können diverse Zusatzstoffe zugesetzt sein, z. B. chlorhaltige Substanzen zur Erhöhung der Sterilisationswirkung und damit Verkürzung der Sterilisationszeit. Dabei ist die damit verbundene Erhöhung der Leitfähigkeit der Flüssigkeit zu beachten und in die Festlegung der erforderlichen elektrischen Feldstärke mit einzubeziehen. Bei einer zu hohen elektrischen Leitfähigkeit der Flüssigkeit kann es zu einem Spannungsdurchschlag kommen, der wiederum zu einer Beeinträchtigung oder Beschädigung des Dialysators und/oder der PEF-Einrichtung führen kann.

An jeder Seite des Dialysators 1 ist über dessen Stirn- bzw. Schnittfläche jeweils eine Elektrode 2 angeordnet und in der PEF (Pulsed Electric Field)-Einrichtung 4 aufgenommen.

In anderen Worten weist der Dialysator 1 an seiner jeweils anderen Stirnseite, die in Fig. 1 nicht dargestellt ist, eine zumindest funktionell spiegelbildliche Anordnung mit einer ersten, oberseitigen Elektrode 2 und einer entsprechenden zweiten, unterseitigen Elektrode (nicht dargestellt) auf. Nachstehend werden daher die Elektroden 2 des Elektrodenpaars am Dialysator 1 im Singular beschrieben, und es versteht sich, dass sich die Beschreibung dennoch auf beide Elektroden 2 des Elektrodenpaars am Dialysator 1 bezieht.

In diesem Ausführungsbeispiel hat die Elektrode 2 eine ringförmige oder (hohl)zylindrische Form, deren Durchmesser vorzugsweise zumindest dem Durchmesser des (mit beispielsweise PUR vergossenen) Hohlfaserbündels im Inneren des Dialysators 1 entspricht, sodass sich bei Anlegen einer elektrischen Spannung an die Elektrode 2 ein homogenes elektrisches Feld innerhalb des Dialysators 1 zwischen der ersten, oberseitigen Elektrode 2 und der zweiten, unterseitigen Elektrode (nicht dargestellt) aufbaut.

Die Elektrode 2 ist jeweils mit einer Isolierung 6 versehen, die beispielsweise eine verbleibende Ausnehmung der PEF-Einrichtung 4 ausfüllt. Zusätzlich weist die Elektrode 2 eine Öffnung, vorzugsweise in Form einer mittigen Bohrung, auf, durch welche während eines Füll- und/oder Spülprozesses Flüssigkeit ein- bzw. ausgebracht werden kann. Die Elektrode 2 und die Isolierung 6 bilden in diesem Ausführungsbeispiel eine Einheit zusammen mit beispielsweise einer äußeren Umhüllung die PEF-Einrichtung 4. Die PEF-Einrichtung 4 kann auch als ein PEF-Adapter ausgeführt sein.

Die PEF-Einrichtung 4 bzw. der PEF-Adapter ermöglicht eine Abdichtung des flüssigkeitsgefüllten Raums zum Gehäuse des Dialysators 1 hin. Dazu weist die PEF-Einrichtung 4 vorzugsweise ein Innengewinde auf, welches bezüglich seiner Auslegung und Dimensionierung dem Gewinde einer Blutkappe für den Dialysator 1 entspricht.

Zu Beginn einer Sterilisationsbehandlung wird jeweils eine PEF-Einrichtung 4 auf beiden (Stirn)Seiten des Dialysators 1 (ohne Blutkappen) aufgeschraubt. Als zusätzliche Dichtungsmaßnahme kann dabei analog zur Abdichtung von Blutkappen ein O-Ring vorgesehen sein. Zweckmäßig kann der Produktionsprozess des Dialysators 1 in der Weise erfolgen, dass die Blutkappenmontage der Sterilisation nachgestellt ist.

In der weiteren Sterilisationsbehandlung wird der Dialysator 1 mit Flüssigkeit gefüllt und nach Erreichen eines vorbestimmten Füllstands von der Flüssigkeitszufuhr abgekoppelt.

Anschließend wird die PEF-Behandlung durchgeführt. Bei der PEF-Behandlung wird der Dialysator 1 mit einer definierten Anzahl elektrischer Pulse mit einer definierten Feldstärke (bzw. Spannung), einer definierten Pulsdauer und einer definierten Pausenzeit zwischen den Pulsen über die Elektroden 2 entlang der Längsrichtung des Dialysators bzw. wird die im Dialysator befindliche Flüssigkeit beaufschlagt.

Die Erzeugung der elektrischen Pulse erfolgt mit an sich bekannter Technik zur Erzeugung hochfrequenter Pulse hoher elektrischer Spannung (z. B. mittels eines Marx-Generators). Die an sich bekannte Technik wird daher an dieser Stelle nicht redundant beschrieben.

Nach einer PEF-Behandlung wie vorstehend beschrieben ist vorzugsweise eine Verweilzeit vorgesehen, in der durch die Spannungspulse erzeugte reaktive Substanzen (reaktive Sauerstoffspezies, elementares Chlor (bei zugesetzten chlorhaltigen Verbindungen zur Flüssigkeit) etc.) wirken können, um die Sterilisationswirkung zu erhöhen. Anschließend wird der Dialysator 1 mit destilliertem bzw. gereinigtem Wasser gespült, um Rückstände wie beispielsweise abgetötete Mikroorganismen, zugesetzte Substanzen und dergleichen zu entfernen.

In Übereinstimmung mit dem ersten Ausführungsbeispiel kann die PEF-Sterilisation beispielsweise wie nachstehend beschrieben in den Produktionsprozess eines Dialysators 1 eingebunden sein.

Nach einleitenden Schritten kann ein Schneiden des Dialysators einschließlich einer Schnittflächenkontrolle erfolgen. Danach kann der Dialysator 1 (ohne Blutkappen) an eine Vorrichtung zur Prüfung der Faserintegrität (nicht gezeigt) angeschlossen werden, und können entsprechende Prüfschritte durchgeführt werden. Sodann kann der Anschluss des Dialysators (ohne Blutkappen) an eine Vorrichtung zur PEF-Sterilisation vorgesehen sein, und kann die Durchführung der Sterilisation erfolgen. Weiter kann danach ein Anschluss des Dialysators 1 (ohne Blutkappen) an eine Vorrichtung zur Trocknung des Dialysators 1 erfolgen, und kann der Dialysator 1 getrocknet werden.

Schließlich werden sterilisierte Blut- und Schutzkappen auf den Dialysator 1 angebracht. Zur Sicherstellung der Sterilität des Blutraums sind die Innenseiten von Blut- und Schutzkappen sowie der O-Ring vorzugsweise oberflächlich zu sterilisieren. Weitere Schritte eines regelmäßigen Produktionsprozesses zur Fertigstellung des Dialysators 1 können sich anschließen.

Bevorzugt können dabei die Prozessschritte des Faserintegritätstests, der PEF-Sterilisation und der Trocknung kombiniert durchgeführt werden.

Darüber hinaus kann die im Dialysator 1 befindliche Flüssigkeit während der PEF-Behandlung umgewälzt werden, um gebildete sterilisierende Substanzen, wie beispielsweise reaktive Sauerstoffspezies, elementares Chlor und dergleichen, gleichmäßig über alle Bereiche und/oder Hohlfasern des Dialysators 1 zu verteilen.

In einer Modifikation kann ferner die PEF-Behandlung intervallweise durchgeführt werden, und können sich dabei Abschnitte bzw. Zeiten oder Intervalle der PEF-Behandlung und Abschnitte bzw. Zeiten oder Intervalle, in welchen die Flüssigkeit umgewälzt wird, abwechseln.

Fig. 2 zeigt schematisch eine Schnittansicht eines Dialysators 1 mit einer PEF-Einrichtung 4, einer Isolierung 6 und einem seitlichen Flüssigkeitszulauf bzw. Flüssigkeitsablauf 3 gemäß einem zweiten Ausführungsbeispiel der Erfindung. Auch der Stopfen 5 im Dialysierflüssigkeitsstutzen ist an einer Seitenwandung des Dialysators 1 vorgesehen.

Das zweite Ausführungsbeispiel des Dialysators 1 mit der PEF-Einrichtung 4 unterscheidet sich von dem des ersten Ausführungsbeispiels dadurch, dass die Flüssigkeit durch einen seitlich unterhalb der Elektrode 2 in eine Öffnung bzw. den Flüssigkeitszulauf bzw. Flüssigkeitsablauf 3 in der Isolierung 6 mündenden Kanal in den Dialysator ein- bzw. ausgebracht werden kann und daher die Elektrode 2 scheibenförmig ausgebildet ist, d.h. keine mittige Bohrung aufweist.

Die vorangehende Beschreibung des ersten Ausführungsbeispiels ist vollumfänglich auch auf das zweite Ausführungsbeispiel anwendbar.

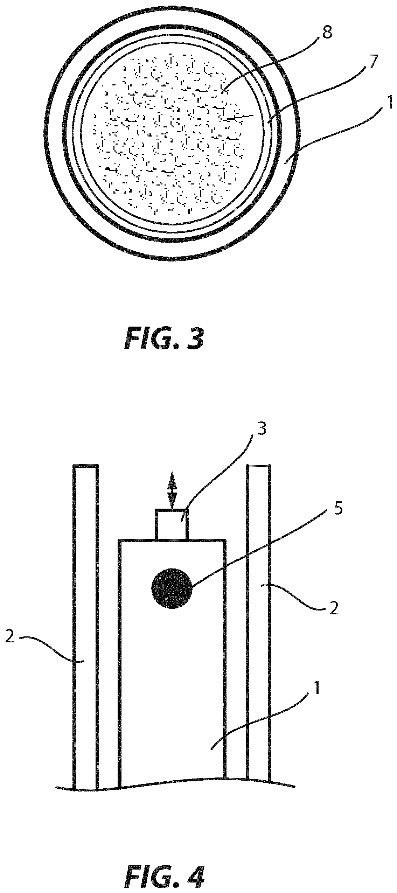

Fig. 3 zeigt schematisch eine Modifikation des ersten und des zweiten Ausführungsbeispiels mit alternativer Abdichtung des flüssigkeitsgefüllten Raums des Dialysators 1 gemäß einem dritten Ausführungsbeispiel.

In dem dritten Ausführungsbeispiel erfolgen die Anbringung der PEF-Einrichtung 4 an dem Dialysator 1 und die Abdichtung des flüssigkeitsgefüllten Raums am Übergang zwischen der PEF-Einrichtung 4 und dem Dialysator 1 anders als bei dem ersten und dem zweiten Ausführungsbeispiel nicht mittels eines Gewindes, sondern durch Aufdrücken oder Aufpressen eines Hohlzylinders (nicht gezeigt) dünner Wandstärke auf den Bereich zwischen Gehäuse und faserhaltiger Schnittfläche 8 des Dialysators 1.

Der Bereich zwischen Gehäuse und faserhaltiger Schnittfläche des Dialysators 1 kann dazu als eine Dichtungsfläche 7 ausgebildet sein, und der Hohlzylinder kann dazu in der PEF-Einrichtung 4 vorgesehen sein.

Die vorangehende Beschreibung des ersten und des zweiten Ausführungsbeispiels ist vollumfänglich auch auf das dritte Ausführungsbeispiel anwendbar.

Fig. 4 zeigt schematisch eine Schnittansicht eines Dialysators 1 mit einer PEF-Einrichtung 4, oberseitigem Flüssigkeitszulauf bzw. Flüssigkeitsablauf und Elektroden 2 in vorbestimmten Abstand außenseitig entlang der Längsseiten des Dialysators 1 gemäß einem vierten Ausführungsbeispiel der Erfindung.

In dem vierten Ausführungsbeispiel sind die Elektroden 2 im Gegensatz zu den ersten bis dritten Ausführungsbeispielen nicht an jeweils den Stirnflächen des Dialysators 1, sondern in Form paralleler Platten an bzw. entlang dessen Längsseiten angeordnet. Die Elektroden 2 erzeugen daher in diesem Ausführungsbeispiel eine Pulsation entlang der Querrichtung des Dialysators 1.

In Simulationen des sich in diesem Fall ausbildenden elektrischen Felds lässt sich zeigen, dass sich bei Elektroden 2 in Form ebener Platten zwischen diesen ein annähernd homogenes elektrisches Feld ausbildet und die elektrische Feldstärke nur in den jeweiligen Randbereichen abnimmt. Diese Abnahme kann jedoch dadurch kompensiert werden, dass die Elektroden 2 geringfügig größer als die Querschnittsfläche des Dialysators 1 dimensioniert werden, wie in Fig. 4 gezeigt.

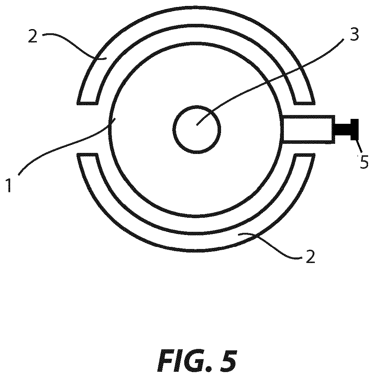

Fig. 5 zeigt schematisch eine Aufsicht auf einen Dialysator 1 mit einer PEF-Einrichtung 4 und gekrümmten Elektroden 2 gemäß einem fünften Ausführungsbeispiel. Das fünfte Ausführungsbeispiel bildet eine Modifikation des vierten Ausführungsbeispiels, bei der die außenseitig angeordneten Elektroden 2 entsprechend dem Umfang und/oder Radius des Dialysators 1 gebogen ausgeformt und in vorbestimmtem Abstand zur Umfangswandung des Dialysators 1 angeordnet sind.

Bei Ausführung der Elektroden 2 in Form gebogener Platten können in dem Raum zwischen den Elektroden 2, in welchem sich der Dialysator 1 befindet, Bereiche mit hoher Feldstärke und Bereiche mit geringerer Feldstärke auftreten. Es ist daher möglich, dass die Sterilisationswirkung nicht gleichmäßig über das gesamte Dialysatorvolumen verteilt wird. Einer derartigen Ungleichverteilung der Sterilisationswirkung kann durch Verringerung der Inhomogenität des elektrischen Felds mit weiter angepasster und/oder spezieller Ausformung der Elektroden 2 und/oder zeitlicher Variation sich örtlich ausbildender Feldmaxima und Feldminima zur Erzeugung einer feldinduzierten Verwirbelung minimierend und/oder kompensierend entgegen gewirkt werden.

Die vorangehende Beschreibung des ersten und des zweiten Ausführungsbeispiels ist vollumfänglich auch auf das vierte und das fünfte Ausführungsbeispiel anwendbar.

Die PEF-Einrichtung 4 gemäß dem ersten bis dritten Ausführungsbeispiel bzw. die Elektroden 2 gemäß dem vierten und dem fünften Ausführungsbeispiel sind derzeit nicht zum dauerhaften Verbleib an einem für praktische Verwendung bestimmten Dialysator vorgesehen, sondern finden während eines Herstellungsprozesses des Dialysators 1 lediglich vorübergehend zu dessen Sterilisation und insbesondere zur Entfernung von bereits im Dialysator 1 vorhandener Keime Anwendung.

Insbesondere genügt daher in dem dritten Ausführungsbeispiel ein Andrücken oder Anpressen der PEF-Einrichtung 4 (Adapter) gegen einen in vorbestimmter Weise abdichtenden Auflagebereich an dem Dialysator 1. Es versteht sich daher, dass in den vorstehend beschriebenen Ausführungsbeispielen Befestigungsvorrichtungen, Andruck- oder Anpressvorrichtungen, Halteeinrichtungen und dergleichen zusätzlich oder alternativ zu jeweils beschriebenen Ausführungsformen fachmännisch vorgesehen sein können, um in durchgeführten Prozessschritten und Ausführungsformen jeweils beteiligte Komponenten an Ort und Stelle und in vorbestimmter Weise fluiddicht zu halten, ohne dass es einer detaillierten Beschreibung solcher Vorrichtungen und Einrichtungen im Einzelnen bedarf.

Außerdem können in dem vierten und in dem fünften Ausführungsbeispiel die plattenförmigen Elektroden 2 bzw. die gekrümmten oder gebogenen Elektroden 2 derart angeordnet und dimensioniert sein, dass während der PEF-Prozessschritte der Dialysator 1 automatisiert zwischen die Elektroden 2 führbar und nach einer vorbestimmten Verweilzeit aus dem Elektrodenraum heraus führbar ist. Gegebenenfalls sind daher vorbestimmte Zwischenräume zwischen den Elektroden 2 oder eine vorbestimmte Positionierung des Dialysators 1 zwischen den Elektroden 2 vorgesehen, wie beispielsweise in Fig. 4 und Fig. 5 durch die Lage des Dialysierflüssigkeitsstutzens bezüglich der Elektroden 2 angedeutet.

Es wird angemerkt, dass mangels physischen Kontakts von PEF-Einrichtung 4 und Flüssigkeit ferner die elektrische Isolation 6 in dem vierten und dem fünften Ausführungsbeispiel gegebenenfalls entfallen kann.

Wie vorstehend beschrieben wurde, beinhaltet eine Vorrichtung zur Sterilisation eines Dialysators für eine extrakorporale Blutbehandlung eine Einrichtung zur Erzeugung eines gepulsten elektrischen Felds, die dazu angeordnet ist, bei Anlegen einer gepulsten elektrischen Spannung zwischen einer ersten Elektrode und einer zweiten Elektrode der Einrichtung ein den zwischen der ersten Elektrode und der zweiten Elektrode aufgenommenen Dialysator durchdringendes gepulstes elektrisches Feld zu erzeugen.

Dabei kann die Einrichtung zur Erzeugung eines gepulsten elektrischen Felds (die PEF-Einrichtung 4) einen ersten Abschnitt, der an einer ersten Stirnseite des Dialysators angeordnet ist, und einen zweiten Abschnitt, der dem ersten Abschnitt gegenüberliegend an einer zweiten Stirnseite des Dialysators angeordnet ist, aufweisen, und können die erste Elektrode in dem ersten Abschnitt und die zweite Elektrode in dem zweiten Abschnitt derart angeordnet sein, dass das gepulste elektrische Feld zumindest ein in dem Dialysator aufgenommenes Hohlfaserbündel in dessen Längsrichtung durchdringt.

Gemäß dem ersten Ausführungsbeispiel wie vorstehend beschrieben können die erste Elektrode und die zweite Elektrode hohlzylinderförmig erzeugt sein, kann der erste Abschnitt der Einrichtung zur Erzeugung eines gepulsten elektrischen Felds einen elektrischen Isolator aufweisen, in dem die erste Elektrode aufgenommen ist, kann der zweite Abschnitt der Einrichtung zur Erzeugung eines gepulsten elektrischen Felds einen elektrischen Isolator aufweisen, in dem die zweite Elektrode aufgenommen ist, und kann durch den ersten Abschnitt und durch den zweiten Abschnitt der Einrichtung zur Erzeugung eines gepulsten elektrischen Felds hindurch jeweils ein in den Dialysator und zur Außenseite der Einrichtung zur Erzeugung eines gepulsten elektrischen Felds hin öffnender Flüssigkeitskanal verlaufen.

Gemäß dem ersten Ausführungsbeispiel kann ferner der in den Dialysator öffnende Flüssigkeitskanal in Richtung der Längsachse des Dialysators durch den Isolator und durch den Hohlzylinder der Elektrode hindurch verlaufen. Der Flüssigkeitskanal kann in diesem Fall durch den Isolator gegenüber der Elektrode isoliert sein.

Gemäß dem zweiten Ausführungsbeispiel wie vorstehend beschrieben können die erste Elektrode und die zweite Elektrode scheibenförmig erzeugt sein, kann der erste Abschnitt der Einrichtung zur Erzeugung eines gepulsten elektrischen Felds einen elektrischen Isolator aufweisen, in dem die erste Elektrode aufgenommen ist, kann der zweite Abschnitt der Einrichtung zur Erzeugung eines gepulsten elektrischen Felds einen elektrischen Isolator aufweisen, in dem die zweite Elektrode aufgenommen ist, und kann durch den ersten Abschnitt und durch den zweiten Abschnitt der Einrichtung zur Erzeugung eines gepulsten elektrischen Felds hindurch jeweils ein in den Dialysator und zur Außenseite der Einrichtung zur Erzeugung eines gepulsten elektrischen Felds hin öffnender Flüssigkeitskanal verlaufen.

Gemäß dem zweiten Ausführungsbeispiel kann ferner in der vorstehenden Alternative der in den Dialysator öffnende Flüssigkeitskanal auf einer ersten Länge in Richtung der Längsachse des Dialysators und auf einer zweiten Länge in Richtung der Querachse des Dialysators zwischen dem Dialysator und der Elektrode durch den Isolator hindurch verlaufen.

Gemäß dem ersten und dem zweiten Ausführungsbeispiel kann die Einrichtung zur Erzeugung eines gepulsten elektrischen Felds mittels einer verschraubbaren Gewindeanordnung an dem Dialysator fluiddicht festlegbar sein.

Gemäß dem dritten Ausführungsbeispiel wie vorstehend beschrieben können alternativ die Einrichtung zur Erzeugung eines gepulsten elektrischen Felds und der Dialysator über eine Dichtfläche, die im Bereich einer Schnittfläche des Hohlfaserbündels das Hohlfaserbündel innenseitig der Wandung des Dialysators umläuft, und eine gegen die Dichtfläche anpressbare Erhebung fluiddicht verbindbar bzw. koppelbar sein.

Gemäß dem vierten Ausführungsbeispiel wie vorstehend beschrieben kann die Einrichtung zur Erzeugung eines gepulsten elektrischen Felds einen ersten Abschnitt und einen zweiten Abschnitt aufweisen, die einander gegenüberliegend entlang der Längsseite des Dialysators angeordnet sind, und kann die erste Elektrode in dem ersten Abschnitt und die zweite Elektrode in dem zweiten Abschnitt derart angeordnet sein, dass das gepulste elektrische Feld zumindest das in dem Dialysator aufgenommene Hohlfaserbündel in dessen Querrichtung durchdringt. Bevorzugt sind in dem vierten Ausführungsbeispiel die erste Elektrode und die zweite Elektrode plattenförmig eben ausgebildet und parallel zueinander angeordnet.

Gemäß dem fünften Ausführungsbeispiel wie vorstehend beschrieben können die erste Elektrode und die zweite Elektrode dem Umfang des Dialysators folgend gekrümmt ausgebildet sein.

In einem Verfahren zur Sterilisation eines Dialysators für eine extrakorporale Blutbehandlung werden in einem Prozess zur Vorbereitung oder Herstellung des Dialysators die folgenden Schritte durchgeführt: Erzeugen eines gepulsten elektrischen Felds mittels einer vorbestimmten Anzahl elektrischer Pulse einer definierten elektrischen Spannung, einer definierten Pulsdauer und einer definierten Pausenzeit zwischen den Pulsen, und Beaufschlagen des Dialysators mit dem gepulsten elektrischen Feld.

Vor dem Erzeugen des gepulsten elektrischen Felds kann dabei eine Einrichtung zur Erzeugung eines gepulsten elektrischen Felds auf jeweils beiden Seiten des Dialysators angeordnet werden, und kann der Dialysator mit einer vorbestimmten Flüssigkeit vor dem Erzeugen des gepulsten elektrischen Felds gefüllt werden. Nach Abschalten oder Wegnehmen des gepulsten elektrischen Felds kann vorgesehen sein, eine vorbestimmte Einwirkungsdauer für durch die elektrischen Pulse erzeugte reaktive Substanzen verstreichen zu lassen, und kann sodann ein Spülen des Dialysators zur Entfernung von Rückständen des Sterilisationsprozesses durchgeführt werden. Bevorzugt wird das vorstehende Verfahren mit einer Vorrichtung einer der vorgenannten Ausführungsformen durchgeführt.

Wie vorstehend beschrieben wurde, umfasst somit eine Vorrichtung zur Sterilisation eines Dialysators für eine extrakorporale Blutbehandlung eine Einrichtung zur Erzeugung eines gepulsten elektrischen Felds, die dazu angeordnet ist, bei Anlegen einer gepulsten elektrischen Spannung zwischen einer ersten Elektrode und einer zweiten Elektrode der Einrichtung ein den zwischen der ersten Elektrode und der zweiten Elektrode aufgenommenen Dialysator durchdringendes gepulstes elektrisches Feld zu erzeugen. Ein unter Verwendung dieser Vorrichtung ausführbares Verfahren zur Sterilisation eines Dialysators für eine extrakorporale Blutbehandlung beinhaltet in einem Prozess zur Vorbereitung oder Herstellung des Dialysators zumindest die Schritte des Erzeugens eines gepulsten elektrischen Felds mittels einer vorbestimmten Anzahl elektrischer Pulse einer definierten elektrischen Spannung, einer definierten Pulsdauer und einer definierten Pausenzeit zwischen den Pulsen, und Beaufschlagens des Dialysators mit dem gepulsten elektrischen Feld.

Die Erfindung wurde vorstehend anhand bevorzugter Ausführungsbeispiele beschrieben. Es versteht sich, dass Einzelheiten der beschriebenen bevorzugten Ausführungsbeispiele die Erfindung als solche nicht beschränken und sich für den Fachmann naheliegende verschiedenartige Änderungen, Modifikationen und/oder Äquivalente ergeben können, die als solche allesamt im Rahmen der durch die beigefügten Ansprüche definierten Schutzbereich der Erfindung liegen.

## Patentansprüche

1. Vorrichtung zur Sterilisation eines Dialysators (1) für eine extrakorporale Blutbehandlung, mit einer Einrichtung zur Erzeugung eines gepulsten elektrischen Felds (4), die dazu angeordnet ist, bei Anlegen einer gepulsten elektrischen Spannung zwischen einer ersten Elektrode (2) und einer zweiten Elektrode (2) der Einrichtung ein den zwischen der ersten Elektrode (2) und der zweiten Elektrode (2) aufgenommenen Dialysator (1) durchdringendes gepulstes elektrisches Feld zu erzeugen, wobei die Einrichtung zur Erzeugung eines gepulsten elektrischen Felds (4) derart angeordnet ist, dass es an physischem Kontakt derselben zu einer in den Dialysator eingebrachten Flüssigkeit mangelt.

2. Vorrichtung nach Anspruch 1, bei der die Einrichtung zur Erzeugung eines gepulsten elektrischen Felds (4) einen ersten Abschnitt, der an einer ersten Stirnseite des Dialysators (1) angeordnet werden kann, und einen zweiten Abschnitt, der dem ersten Abschnitt gegenüberliegend an einer zweiten Stirnseite des Dialysators (1) angeordnet werden kann, aufweist, und die erste Elektrode (2) in dem ersten Abschnitt und die zweite Elektrode (2) in dem zweiten Abschnitt derart angeordnet werden können, dass das gepulste elektrische Feld zumindest ein in dem Dialysator (1) aufgenommenes Hohlfaserbündel (8) in dessen Längsrichtung durchdringt.

3. Vorrichtung nach Anspruch 2, bei der die erste Elektrode (2) und die zweite Elektrode (2) hohlzylinderförmig erzeugt sind, der erste Abschnitt der Einrichtung zur Erzeugung eines gepulsten elektrischen Felds (4) einen elektrischen Isolator (6) aufweist, in dem die erste Elektrode (2) aufgenommen ist, der zweite Abschnitt der Einrichtung zur Erzeugung eines gepulsten elektrischen Felds (4) einen elektrischen Isolator (6) aufweist, in dem die zweite Elektrode (2) aufgenommen ist, und durch den ersten Abschnitt und durch den zweiten Abschnitt der Einrichtung zur Erzeugung eines gepulsten elektrischen Felds (4) hindurch jeweils ein in den Dialysator (1) und zur Außenseite der Einrichtung zur Erzeugung eines gepulsten elektrischen Felds (4) hin öffnender Flüssigkeitskanal (3) verläuft.

4. Vorrichtung nach Anspruch 3, bei der der in den Dialysator (1) öffnende Flüssigkeitskanal (3) in Richtung der Längsachse des Dialysators (1) durch den Isolator (6) und durch den Hohlzylinder der Elektrode (2) hindurch verläuft, wobei der Flüssigkeitskanal (3) durch den Isolator (6) gegenüber der Elektrode (2) isoliert ist.

5. Vorrichtung nach Anspruch 2, bei der die erste Elektrode (2) und die zweite Elektrode (2) scheibenförmig erzeugt sind, der erste Abschnitt der Einrichtung zur Erzeugung eines gepulsten elektrischen Felds (4) einen elektrischen Isolator (6) aufweist, in dem die erste Elektrode (2) aufgenommen ist, der zweite Abschnitt der Einrichtung zur Erzeugung eines gepulsten elektrischen Felds (4) einen elektrischen Isolator (6) aufweist, in dem die zweite Elektrode (2) aufgenommen ist, und durch den ersten Abschnitt und durch den zweiten Abschnitt der Einrichtung zur Erzeugung eines gepulsten elektrischen Felds (4) hindurch jeweils ein in den Dialysator (1) und zur Außenseite der Einrichtung zur Erzeugung eines gepulsten elektrischen Felds (4) hin öffnender Flüssigkeitskanal (3) verläuft.

6. Vorrichtung nach Anspruch 5, bei der der in den Dialysator (1) öffnende Flüssigkeitskanal (3) auf einer ersten Länge in Richtung der Längsachse des Dialysators (1) und auf einer zweiten Länge in Richtung der Querachse des Dialysators (1) zwischen dem Dialysator (1) und der Elektrode (2) durch den Isolator (6) hindurch verläuft.

7. Vorrichtung nach einem der vorangehenden Ansprüche, bei der die Einrichtung zur Erzeugung eines gepulsten elektrischen Felds (4) mittels einer verschraubbaren Gewindeanordnung an dem Dialysator (1) fluiddicht festlegbar ist.

8. Vorrichtung nach einem der vorangehenden Ansprüche 1 bis 6, bei der die Einrichtung zur Erzeugung eines gepulsten elektrischen Felds (4) und der Dialysator (1) über eine Dichtfläche (7), die im Bereich einer Schnittfläche des Hohlfaserbündels (8) das Hohlfaserbündel (8) innenseitig der Wandung des Dialysators (1) umläuft, und eine gegen die Dichtfläche (7) anpressbare Erhebung fluiddicht verbindbar sind.

9. Vorrichtung nach Anspruch 1, bei der die Einrichtung zur Erzeugung eines gepulsten elektrischen Felds (4) einen ersten Abschnitt und einen zweiten Abschnitt, die einander gegenüberliegend entlang der Längsseite des Dialysators (1) angeordnet sind, aufweist, und die erste Elektrode (2) in dem ersten Abschnitt und die zweite Elektrode (2) in dem zweiten Abschnitt derart angeordnet werden können, dass das gepulste elektrische Feld zumindest das in dem Dialysator (1) aufgenommene Hohlfaserbündel (8) in dessen Querrichtung durchdringt.

10. Vorrichtung nach Anspruch 9, bei der die erste Elektrode (2) und die zweite Elektrode (2) plattenförmig eben ausgebildet und parallel zueinander angeordnet sind.

11. Vorrichtung nach Anspruch 9, bei der die erste Elektrode (2) und die zweite Elektrode (2) dem Umfang des Dialysators (1) folgend gekrümmt ausgebildet sind.

12. Verfahren zur Sterilisation eines Dialysators für eine extrakorporale Blutbehandlung, mit folgenden Schritten in einem Prozess zur Vorbereitung oder Herstellung des Dialysators (1):
- Erzeugen eines gepulsten elektrischen Felds durch Elektroden (2) einer Einrichtung (4) zur Erzeugung eines gepulsten elektrischen Felds, wobei die Einrichtung (4) derart angeordnet ist, dass es an physischem Kontakt derselben zu einer in den Dialysator eingebrachten Flüssigkeit mangelt, mittels einer vorbestimmten Anzahl elektrischer Pulse einer definierten elektrischen Spannung, einer definierten Pulsdauer und einer definierten Pausenzeit zwischen den Pulsen, und
- Beaufschlagen des Dialysators (1) mit dem gepulsten elektrischen Feld.

13. Verfahren nach Anspruch 12, ferner mit den Schritten:
- vor dem Erzeugen des gepulsten elektrischen Felds:
- Anordnen einer Einrichtung zur Erzeugung eines gepulsten elektrischen Felds (4) auf jeweils beiden Seiten des Dialysators (1); und
- Füllen des Dialysators (1) mit einer vorbestimmten Flüssigkeit vor dem Erzeugen des gepulsten elektrischen Felds; und
- nach Wegnahme des gepulsten elektrischen Felds:
- Verstreichen lassen einer vorbestimmten Einwirkungsdauer für durch die elektrischen Pulse erzeugte reaktive Substanzen; und
- Spülen des Dialysators (1) zur Entfernung von Rückständen des Sterilisationsprozesses.

14. Verfahren nach Anspruch 12 oder 13, das mit einer Vorrichtung nach einem der Ansprüche 1 bis 11 durchgeführt wird.

## Claims

1. An apparatus for sterilizing a dialyzer (1) for extracorporeal blood treatment, comprising means for generating a pulsed electric field (4) which is arranged for generating a pulsed electric field penetrating the dialyzer (1) received between the first electrode (2) and the second electrode (2), when a pulsed electric voltage is applied between a first electrode (2) and a second electrode (2) of the means, wherein the means for generating a pulsed electric field (4) is arranged such that there is a lack of liquid inserted into the dialyzer at the physical contact thereof.

2. The apparatus according to claim 1, wherein the means for generating a pulsed electric field (4) includes a first section adapted to be disposed on a first end face of the dialyzer (1) and a second section adapted to be disposed opposite to the first section on a second end face of the dialyzer (1), and the first electrode (2) can be arranged in the first section and the second electrode (2) can be arranged in the second section so that the pulsed electric field penetrates at least one hollow fiber bundle (8) accommodated in the dialyzer (1) in the longitudinal direction thereof.

3. The apparatus according to claim 2, wherein the first electrode (2) and the second electrode (2) are produced in hollow cylindrical shape, the first section of the means for generating a pulsed electric field (4) includes an electric insulator (6) in which the first electrode (2) is received, the second section of the means for generating a pulsed electric field (4) includes an electric insulator (6) in which the second electrode (2) is received, and a liquid channel (3) opening into the dialyzer (1) and toward the outside of the means for generating a pulsed electric field (4) extends through each of the first section and the second section of the means for generating a pulsed electric field (4).

4. The apparatus according to claim 3, wherein the liquid channel (3) opening into the dialyzer (1) extends in the direction of the longitudinal axis of the dialyzer (1) through the insulator (6) and through the hollow cylinder of the electrode (2), wherein the liquid channel (3) is insulated against the electrode (2) by the insulator (6).

5. The apparatus according to claim 2, wherein the first electrode (2) and the second electrode (2) are produced in disk shape, the first section of the means for generating a pulsed electric field (4) includes an electric insulator (6) in which the first electrode (2) is received, the second section of the means for generating a pulsed electric field (4) includes an electric insulator (6) in which the second electrode (2) is received, and a liquid channel (3) opening into the dialyzer (1) and toward the outside of the means for generating a pulsed electric field (4) extends through each of the first section and the second section of the means for generating a pulsed electric field (4).

6. The apparatus according to claim 5, wherein the liquid channel (3) opening into the dialyzer (1) extends along a first length in the direction of the longitudinal axis of the dialyzer (1) and along a second length in the direction of the transverse axis of the dialyzer (1) between the dialyzer (1) and the electrode (2) through the insulator (6).

7. The apparatus according to claim 1, wherein the means for generating a pulsed electric field (4) can be fixed to the dialyzer (1) in a fluid-tight manner via a screw-on thread arrangement.

8. The apparatus according to claim 1, wherein the means for generating a pulsed electric field (4) and the dialyzer (1) can be connected in a fluid-tight manner via a sealing face (7) surrounding the hollow fiber bundle (8) in the area of a cut face of the hollow fiber bundle (8) on the inside of the wall of the dialyzer (1) and via an elevation adapted to be pressed against the sealing face (7).

9. The apparatus according to claim 1, wherein the means for generating a pulsed electric field (4) includes a first section and a second section which are arranged opposite to each other along the longitudinal side of the dialyzer (1), and the first electrode (2) can be arranged in the first section and the second electrode (2) can be arranged in the second section so that the pulsed electric field penetrates at least the hollow fiber bundle (8) accommodated in the dialyzer (1) in the transverse direction thereof.

10. The apparatus according to claim 9, wherein the first electrode (2) and the second electrode (2) are flatly plate-shaped and are arranged in parallel to each other.

11. The apparatus according to claim 9, wherein the first electrode (2) and the second electrode (2) are curved following the circumference of the dialyzer (1).

12. A method of sterilizing a dialyzer for extracorporeal blood treatment, comprising the following steps in a process for preparing or manufacturing the dialyzer (1):
- generating a pulsed electric field, by electrodes (2) of a means (4) for generating a pulsed electric field wherein the means (4) is arranged in such a way that there is a lack of liquid inserted into the dialyzer at the physical contact thereof, via a predetermined number of electric pulses of a defined electric voltage, a defined pulse duration and a defined pulse-off time between the pulses, and
- applying the pulsed electric field to the dialyzer (1).

13. The method according to claim 12, further comprising the steps of:
- prior to generating the pulsed electric field:
- arranging a means for generating a pulsed electric field (4) on both sides of the dialyzer (1); and
- filling the dialyzer (1) with a predetermined liquid prior to generating the pulsed electric field; and
- after removing the pulsed electric field:
- allowing a predetermined exposure time for reactive substances generated by the electric pulses to expire; and
- flushing the dialyzer (1) to remove residues of the sterilization process.

14. The method according to claim 12 or 13 which is carried out by an apparatus according to any one of the claims 1 to 11.

## Revendications

1. Dispositif de stérilisation d'un dialyseur (1) pour un traitement extracorporel du sang, avec un appareil destiné à produire un champ électrique pulsé (4) qui est agencé pour produire un champ électrique pulsé traversant le dialyseur (1) reçu entre la première électrode (2) et la seconde électrode (2) lors de l'application d'une tension électrique pulsée entre une première électrode (2) et une seconde électrode (2) de l'appareil, dans lequel l'appareil destiné à produire un champ électrique pulsé (4) est agencé de telle sorte qu'il n'y ait aucun contact physique entre celui-ci et un liquide introduit dans le dialyseur.

2. Dispositif selon la revendication 1, pour lequel l'appareil destiné à produire un champ électrique pulsé (4) présente une première section, qui peut être agencée sur un premier côté frontal du dialyseur (1), et une seconde section, qui peut être agencée à l'opposé de la première section sur un second côté frontal du dialyseur (1), et la première électrode (2) peut être agencée dans la première section et la seconde électrode (2) dans la seconde section de telle sorte que le champ électrique pulsé traverse au moins un faisceau de fibres creuses (8) reçu dans le dialyseur (1) dans sa direction longitudinale.

3. Dispositif selon la revendication 2, pour lequel la première électrode (2) et la seconde électrode (2) sont produites en forme de cylindre creux, la première section de l'appareil destiné à produire un champ électrique pulsé (4) présente un isolant électrique (6) dans lequel la première électrode (2) est reçue, la seconde section de l'appareil destiné à produire un champ électrique pulsé (4) présente un isolant électrique (6) dans lequel la seconde électrode (2) est reçue, et respectivement un canal de liquide (3) s'ouvrant dans le dialyseur (1) et vers le côté extérieur de l'appareil destiné à produire un champ électrique pulsé (4) s'étend à travers la première section et à travers la seconde section de l'appareil destiné à produire un champ électrique pulsé (4).

4. Dispositif selon la revendication 3, pour lequel le canal de liquide (3) s'ouvrant dans le dialyseur (1) dans la direction de l'axe longitudinal du dialyseur (1) s'étend à travers l'isolant (6) et à travers le cylindre creux de l'électrode (2), dans lequel le canal de liquide (3) est isolé de l'électrode (2) par l'isolant (6).

5. Dispositif selon la revendication 2, pour lequel la première électrode (2) et la seconde électrode (2) sont produites en forme de disque, la première section de l'appareil destiné à produire un champ électrique pulsé (4) présente un isolant électrique (6) dans lequel la première électrode (2) est reçue, la seconde section de l'appareil destiné à produire un champ électrique pulsé (4) présente un isolant électrique (6) dans lequel la seconde électrode (2) est reçue, et respectivement un canal de liquide (3) s'ouvrant dans le dialyseur (1) et vers le côté extérieur de l'appareil destiné à produire un champ électrique pulsé (4) s'étend à travers la première section et à travers la seconde section de l'appareil destiné à produire un champ électrique pulsé (4).

6. Dispositif selon la revendication 5, pour lequel le canal de liquide (3) s'ouvrant dans le dialyseur (1) s'étend sur une première longueur dans la direction de l'axe longitudinal du dialyseur (1) et sur une seconde longueur dans la direction de l'axe transversal du dialyseur (1) entre le dialyseur (1) et l'électrode (2) à travers l'isolant (6).

7. Dispositif selon l'une quelconque des revendications précédentes, pour lequel l'appareil destiné à produire un champ électrique pulsé (4) peut être fixé de manière étanche aux liquides sur le dialyseur (1) au moyen d'un agencement fileté pouvant être vissé.

8. Dispositif selon l'une quelconque des revendications précédentes 1 à 6, pour lequel l'appareil destiné à produire un champ électrique pulsé (4) et le dialyseur (1) peuvent être reliés de manière étanche aux liquides par le biais d'une surface d'étanchéité (7), qui dans la zone d'une surface de coupe du faisceau de fibres creuses (8) entoure le faisceau de fibres creuses (8) du côté intérieur de la paroi du dialyseur (1), et d'une élévation pouvant être pressée contre la surface d'étanchéité (7).

9. Dispositif selon la revendication 1, pour lequel l'appareil destiné à produire un champ électrique pulsé (4) présente une première section et une seconde section qui sont agencées de manière opposée l'une à l'autre le long du côté longitudinal du dialyseur (1), et la première électrode (2) peut être agencée dans la première section et la seconde électrode (2) dans la seconde section de telle sorte que le champ électrique pulsé traverse au moins le faisceau de fibres creuses (8) reçu dans le dialyseur (1) dans sa direction transversale.

10. Dispositif selon la revendication 9, pour lequel la première électrode (2) et la seconde électrode (2) sont conçues en forme de plaque plate et sont agencées parallèlement l'une à l'autre.

11. Dispositif selon la revendication 9, pour lequel la première électrode (2) et la seconde électrode (2) sont conçues de manière incurvée en suivant la périphérie du dialyseur (1).

12. Procédé de stérilisation d'un dialyseur destiné à un traitement extracorporel du sang comprenant les étapes suivantes lors d'un processus de préparation ou de fabrication du dialyseur (1) :
- production d'un champ électrique pulsé par des électrodes (2) d'un appareil (4) destiné à produire un champ électrique pulsé, dans lequel l'appareil (4) est agencé de telle sorte qu'il n'y ait aucun contact physique entre celui-ci et un liquide introduit dans le dialyseur, au moyen d'un nombre prédéterminé d'impulsions électriques d'une tension électrique définie, d'une durée d'impulsion définie et d'un temps de pause défini entre les impulsions, et
- alimentation du dialyseur (1) avec le champ électrique pulsé.

13. Procédé selon la revendication 12, comprenant en outre les étapes suivantes :
- avant la production du champ électrique pulsé :
- agencer un appareil destiné à produire un champ électrique pulsé (4) sur respectivement les deux côtés du dialyseur (1) ; et
- remplir le dialyseur (1) avec un liquide prédéterminé avant la production du champ électrique pulsé ; et
- après le retrait du champ électrique pulsé :
- laisser s'écouler une durée d'action prédéterminée pour les substances réactives produites par les impulsions électriques ; et
rincer le dialyseur (1) pour éliminer les résidus du processus de stérilisation.

14. Procédé selon la revendication 12 ou 13 qui est réalisé avec un dispositif selon l'une quelconque des revendications 1 à 11.
